# EUROPÄISCHE PATENTSCHRIFT

(11) **EP 0 593 524 B1**
(45) Veröffentlichungstag und Bekanntmachung des Hinweises auf die Patenterteilung: **13.12.1995**
(21) Anmeldenummer: 92912840.3
(22) Anmeldetag: 29.06.1992
(51) Int. Cl.: C11C 3/04, B01J 31/04

(54) **VERFAHREN ZUR HERSTELLUNG VON FETTSÄURENIEDRIGALKYLESTERN**
METHOD OF PREPARING LOWER-ALKYL ESTERS OF FATTY ACIDS
PROCEDE DE FABRICATION D'ALKYLESTERS INFERIEURS D'ACIDES GRAS

(30) Priorität: 08.07.1991 DE 4122530
(43) Veröffentlichungstag der Anmeldung: 27.04.1994
(73) Patentinhaber: Henkel Kommanditgesellschaft auf Aktien, 40191 Düsseldorf (DE)
(72) Erfinder: DEMMERING, Günther, D-5650 Solingen (DE); PELZER, Christian, D-5172 Linnich (DE); FRIESENHAGEN, Lothar, D-4000 Düsseldorf (DE)
(86) Internationale Anmeldenummer: EP9201463
(87) Internationale Veröffentlichungsnummer: WO9301263

(56) Entgegenhaltungen:
- EP-A- 0 127 104
- EP-A- 0 198 243
- DE-A- 2 824 782
- WORLD PATENTS INDEX Section Ch, Week 7813, Derwent Publications Ltd., London, GB; Class D, AN 78-24527A & JP,A,53 006 161

## Beschreibung

### Gebiet der Erfindung

Die Erfindung betrifft ein verfahren zur Herstellung von Fettsäureniedrigalkylestern durch Umesterung von Fettsäureglyceriden mit niederen aliphatischen Alkoholen in Gegenwart von freien Fettsäuren.

### Stand der Technik

Fettsäuremethylester stellen wichtige industrielle Rohstoffe für die Herstellung einer Vielzahl von Produkten, beispielsweise Schmierstoffen und Tensiden dar. Üblicherweise geht man zur Herstellung der Ester von natürlichen Fetten und Ölen, also voll- oder Partialestern des Glycerins mit Fettsäuren, aus, die in Gegenwart von Katalysatoren mit Methanol umgeestert werden. Verfahren, die die Umesterung von Fetten und Ölen zum Gegenstand haben, sind in einer vielzahl von Publikationen beschrieben; stellvertretend soll an dieser Stelle auf den Überblicksartikel in **Seifen-Öle-Fette-Wachse, 114**, **595 (1988)** verwiesen werden.

Um die Umesterung in einem aus wirtschaftlicher Sicht rentablen Zeitraum mit befriedigenden Umsetzungsgraden durchführen zu können, müssen Katalysatoren eingesetzt werden. Hierfür kommen insbesondere Schwermetallverbindungen, wie beispielsweise Zinkoxid [**GB 71 27 47**) oder Zinksilicat [**US 2 727 049**] in Betracht. Derartige Verfahren sind jedoch mit dem Nachteil behaftet, daß die Katalysatoren nach der Umesterung aus toxikologischen Gründen nicht im Produkt verbleiben können, sondern mit hohem technischen Aufwand abgetrennt werden müssen.

Schließlich ist aus der EP-A 0198243 ein Verfahren zur Herstellung von C₆-C₂₄ Fettsäurealkylestern durch Umsetzung Triglyceriden, Partialglyceriden und/oder entsprechenden Fettsäuren mit C₁-C₄ Alkoholen in Gegenwart von Aluminiumoxid-Katalysatoren bekannt.

Die Umesterung kann auch in Gegenwart von Mineralsäuren durchgeführt werden, die nach Abschluß der Reaktion lediglich neutralisiert werden müssen. Doch obschon das Problem der Abtrennung hierbei entfällt, sind die Raum-Zeit-Ausbeuten an Umesterungsprodukten im Vergleich zur Schwermetallkatalyse deutlich schlechter, was eine technische Anwendbarkeit des Verfahrens relativiert.

Die Aufgabe der Erfindung bestand somit darin, ein Verfahren zur Umesterung von Fettsäureglyceriden zu entwickeln, das frei von den geschilderten Nachteilen ist.

### Beschreibung der Erfindung

Gegenstand der Erfindung ist ein Verfahren zur Herstellung von Fettsäureniedrigalkylestern durch Umesterung von Fettsäureglyceriden in Gegenwart saurer Katalysatoren, das sich dadurch auszeichnet, daß man Voll- und/oder Partialester des Glycerins mit Fettsäuren der Formel **(I)**,

**R¹-COOH (I)**

in der R¹ für einen aliphatischen Kohlenwasserstoffrest mit 5 bis 23 Kohlenstoffatomen und 0 oder 1 bis 5 Doppelbindungen steht, in Gegenwart mindestens einer Fettsäure der Formel **(II)**,

**R²-COOH (II)**

in der R² für einen aliphatischen Kohlenwasserstoffrest mit 11 bis 17 Kohlenstoffatomen und 0, 1 oder 2 Doppelbindungen steht, bei erhöhter Temperatur und gegebenenfalls unter erhöhtem Druck mit niederen aliphatischen Alkoholen mit 1 bis 4 Kohlenstoffatomen umsetzt.

Überraschenderweise wurde gefunden, daß die Umesterung von Fettsäureglyceriden in Gegenwart von Fettsäuren zu Raum-Zeit-Ausbeuten an Umesterungsprodukten führt, wie sie bislang nur in Gegenwart von Schwermetallverbindungen zu erreichen gewesen sind. Ein weiterer Vorteil des Verfahrens liegt darin, daß die Fettsäure nach Abschluß der Umesterung ebenfalls als Ester vorliegt, eine Abtrennung beziehungsweise Neutralisation des Katalysators somit entfällt. Ferner lassen sich Anlagenstillstände als Folge von Katalysatorablagerungen reduzieren und eine Belastung des freigesetzten Glycerins und des Abwassers durch Schwermetallspuren vermeiden.

Als Einsatzstoffe für die Umesterung kommen im Sinne des erfindungsgemäßen Verfahrens sowohl voll- als auch Partialester des Glycerins mit Fettsäuren in Betracht; bevorzugt ist der Einsatz von Triglyceriden. Typische Beispiele sind Glycerinester der Capronsäure, Caprylsäure, Caprinsäure, Laurinsäure, Myristylsäure, Palmitinsäure, Palmitoleinsäure, Stearinsäure, Ölsäure, Elaidinsäure, Petroselinsäure, Linolsäure, Linolensäure, Chaulmoograsäure, Ricinolsäure, Arachinsäure, Gadoleinsäure, Behensäure, Erucasäure, Arachidonsäure und Clupanodonsäure.

Bei den Glycerinestern der genannten Fettsäuren kann es sich sowohl um synthetische, als auch natürliche Produkte handeln, insbesondere auch solche, bei denen das Glycerin mit zwei oder drei verschiedenen Fettsäuren verknüpft ist. Unter Glycerinfettsäureestern auf natürlicher Basis sind Fette und Öle tierischer oder pflanzlicher Herkunft zu verstehen, beispielsweise Kokosöl, Palmöl, Palmkernöl, Erdnußöl, Baumwollsaatöl, Rapsöl, Sonnenblumenöl, Korianderöl, Leinöl, Sojaöl, Rindertalg oder Fischöl.

Die natürlichen Fettsäureglyceride können ohne weitere Vorbehandlung in die Umesterung eingesetzt werden. Es empfiehlt sich jedoch, die Einsatzstoffe zuvor einer Behandlung mit Bleicherde ("Fullern") zu unterziehen, um auf diesem Wege eine Abtrennung von Schalenresten und Schleimstoffen zu erreichen.

Als niedere aliphatische Alkohole, die gegen das Glycerin im Fettsäureester ausgetauscht werden, kommen Ethanol, n-Propylalkohol, i-Propylalkohol, n-Butanol oder tert.Butanol in Betracht. Bevorzugt wird die Umesterung mit Methanol durchgeführt.

Typische Beispiele für Fettsäuren der Formel **(II)**, die im Sinne des erfindungsgemäßen Verfahrens die Umesterung der Fettsäureglyceride katalysieren, sind Laurinsäure, Myristinsäure, Palmitinsäure, Stearinsäure, Ölsäure und Linolsäure.

Wie in der Fettchemie üblich, können diese Fettsäuren auch in Form technischer Schnitte vorliegen, wie sie bei der Druckspaltung von natürlichen Fetten und Ölen, beispielsweise Palmöl, Palmkernöl, Kokosöl oder Rindertalg anfallen. Bevorzugt ist die Verwendung von technischen Kokosfettsäuren, technischen Palmkernfettsäuren, technischer Ölsäure sowie deren Gemische.

Die Menge an Fettsäuren der Formel **(II)**, die den Glyceriden als Katalysator zugesetzt werden, richtet sich nach der Säurezahl der Einsatzprodukte. Es hat sich als optimal erwiesen, die Säurezahl der Reaktionsmischung, enthaltend das Glycerid und den Alkohol, durch Zugabe von freien Fettsäuren auf eine Säurezahl von 10 bis 30, vorzugsweise 15 bis 20 und insbesondere 18 bis 19 einzustellen. Weisen die Mischungen niedrigere Säurezahlen auf, werden in der Umesterung nur unbefriedigende Ausbeuten erzielt. Eine zu hohe Säurezahl der Reaktionsmischung führt dazu, daß auch im umgeesterten Produkt noch freie, nichtveresterte Fettsäuren enthalten sind, die nachträglich neutralisiert werden müssen.

Werden anstelle entsäuerter Öle unbehandelte Fettsäureglyceride eingesetzt, die noch Säurezahlen unterhalb von 10 aufweisen, ist im Sinne des erfindungsgemäßen Verfahrens die Menge an Fettsäure so auszuwählen, daß sich in der Reaktionsmischung eine Gesamt-Säurezahl von 10 bis 30 einstellt.

Üblicherweise kann dabei die Menge an katalytisch wirksamer Fettsäure der Formel **(II)** 1 bis 20, vorzugsweise 2 bis 15 und insbesondere 3 bis 10 Gew.-% - bezogen auf das Fettsäureglycerid - betragen.

Die Fettsäuren der Formel **(II)** können einem der beiden Einsatzstoffe - Fettsäureglycerid oder Alkohol - oder aber deren Mischung, beispielsweise durch Einrühren bei gegebenenfalls erhöhter Temperatur, zudosiert werden. Die Umesterung kann in an sich bekannter Weise bei erhöhter Temperatur und gegebenenfalls unter erhöhtem Druck, beispielsweise bei Temperaturen von 150 bis 300, insbesondere 200 bis 250°C und Drücken von 1 bis 100, insbesondere 50 bis 80 bar durchgeführt werden. An die Umesterung kann sich eine Aufarbeitung anschließen, bei der das freigesetzte Glycerin und Reste nicht umgesetzten Alkohols abgetrennt werden und der erhaltene Fettsäureniedrigalkylester destilliert beziehungsweise fraktioniert wird.

Die folgenden Beispiele sollen den Gegenstand der Erfindung näher erläutern, ohne ihn darauf einzuschränken.

### Beispiele

### Beispiel 1:

In einem 1-l-Autoklaven wurden 320 g (0,5 mol) raffiniertes Kokosöl der Fettsäure-Zusammensetzung

| | |
|---|---|
| Capronsäure | 0,5 Gew.-% |
| Caprylsäure | 8 Gew.-% |
| Caprinsäure | 7 Gew.-% |
| Laurinsäure | 48 Gew.-% |
| Myristinsäure | 17 Gew.-% |
| Palmitinsäure | 9 Gew.-% |
| Stearinsäure | 2 Gew.-% |
| Ölsäure | 7 Gew.-% |
| Linolsäure | 1,5 Gew.-% |
| Säurezahl | 0,3 |

mit 320 g (10 mol) Methanol (Volumenverhältnis 1 : 1) vorgelegt und mit 58 g - entsprechend 9 Gew.-% bezogen auf die Einsatzprodukte - technischer Ölsäure (mittleres Molgewicht 281) versetzt. Für die Mischung wurden die folgenden Kennzahlen bestimmt:

| | |
|---|---|
| Gebundenes Glycerin | 13,8 Gew.-% |
| Säurezahl | 18 |

Anschließend wurde die Reaktionsmischung über einen Zeitraum von 180 min bei einer Temperatur von 240°C und einem Druck von 65 bar gehalten. Nach dem Abkühlen und Entspannen der Reaktionsmischung wurde ein Umesterungsprodukt erhalten, das die folgenden Kennzahlen aufwies:

| | |
|---|---|
| Gebundenes Glycerin | 1,2 Gew.-% |
| Säurezahl | 0,8 |

### Beispiel 2:

Beispiel 1 wurde mit 320 g unraffiniertem, jedoch zuvor mit Bleicherde und Aktivkohle im Massenverhältnis 1 : 1 behandeltem Kokosöl (Säurezahl : 9,0) und 320 g Methanol wiederholt. Als Umesterungskatalysator wurden diesmal 20,5 g -entsprechend 3 Gew.-% bezogen auf die Einsatzprodukte - einer technischen C₁₂/₁₈-Fettsäure (mittleres Molgewicht 200) auf Basis Kokosöl/Palmkernöl (1:1) eingesetzt.

| | | |
|---|---|---|
| Gebundenes Glycerin | (vor der Umesterung) | 13,8 Gew.-% |
| Säurezahl | (vor der Umesterung) | 18 |
| Gebundenes Glycerin | (nach der Umesterung) | 1,0 Gew.-% |
| Säurezahl | (nach der Umesterung) | 0,8 |

### Beispiel 3:

Beispiel 1 wurde mit 320 g (0,5 mol) unraffiniertem, jedoch zuvor mit Bleicherde und Aktivkohle im Massenverhältnis 1 : 1 behandeltem Palmkernöl der Fettsäure-Zusammensetzung

| | |
|---|---|
| Capronsaure | 0,5 Gew.-% |
| Caprylsäure | 4 Gew.-% |
| Caprinsäure | 5 Gew.-% |
| Laurinsäure | 51 Gew.-% |
| Myristinsäure | 15 Gew.-% |
| Palmitinsäure | 7 Gew.-% |
| Stearinsäure | 2 Gew.-% |
| Ölsäure | 15 Gew.-% |
| Linolsäure | 0,5 Gew.-% |
| Säurezahl | 0,8 |

und 320 g Methanol wiederholt. Als Umesterungskatalysator wurden diesmal 47 g - entsprechend 7,3 Gew.-% bezogen auf die Einsatzprodukte - eines Gemisches einer technischen C₁₂/₁₈-Fettsäure (mittleres Molgewicht 200) und einer technischen Ölsäure (mittleres Molgewicht 281) im Gewichtsverhältnis 1 : 1 eingesetzt.

| | | |
|---|---|---|
| Gebundenes Glycerin | (vor der Umesterung) | 13,2 Gew.-% |
| Säurezahl | (vor der Umesterung) | 18 |
| Gebundenes Glycerin | (nach der Umesterung) | 1,4 Gew.-% |
| Säurezahl | (nach der Umesterung) | 1,1 |

### Beispiel 4:

Die Umesterung wurde kontinuierlich in einer Apparatur durchgeführt, die wie folgt aufgebaut war: Aus Ansaugbehältern für das Methanol bzw. das Öl/Fettsäure-Gemisch wurden die Einsatzstoffe mit Hilfe von Presspumpen über eine Vorheizung kontinuierlich in den Reaktor gefordert. Nach der Umesterung wurde das rohe Reaktionsgemisch abgekühlt und über einen Druckabscheider und ein Druckhalteventil in ein Entspannungsgefäß gefördert.

In der Anlage wurde eine Mischung aus 5000 g (7,8 mol) raffiniertem Kokosöl der Fettsäurezusammensetzung

| | |
|---|---|
| Capronsäure | 0,5 Gew.-% |
| Caprylsäure | 8 Gew.-% |
| Caprinsäure | 7 Gew.-% |
| Laurinsäure | 48 Gew.-% |
| Myristinsäure | 17 Gew.-% |
| Palmitinsäure | 9 Gew.-% |
| Stearinsäure | 2 Gew.-% |
| Ölsäure | 7 Gew.-% |
| Linolsäure | 1,5 Gew.-% |
| Säurezahl | 0,3 |

und 450 g - entsprechend 9 Gew.-% - einer technischen C₁₂/₁₈-Kokosfettsäure (mittleres Molgewicht 200) mit Methanol im Volumenverhältnis 1 : 1,3 zur Reaktion gebracht. Der Durchsatz an Öl/Fettsäure betrug 0,9 l/h, der Durchsatz an Methanol 1,2 l/h.

Nach einer Reaktionszeit von ca. 70 min bei einer Temperatur von 240°C und einem Druck von 9 bar fiel das Umesterungsgemisch nach Abkühlen und Entspannen als zweiphasiges Produkt an.

| | | |
|---|---|---|
| Gebundenes Glycerin | (vor der Umesterung) | 13,8 Gew.-% |
| Säurezahl | (vor der Umesterung) | 18 |
| Gebundenes Glycerin | (nach der Umesterung) | 1,4 Gew.-% |
| Säurezahl | (nach der Umesterung) | 1,5 |

## Patentansprüche

1. Verfahren zur Herstellung von Fettsäureniedrigalkylestern, bei dem man
a) Voll- und/oder Partialester des Glycerins mit Fettsäuren der Formel **(I)**,
**R¹-COOH (I)**
in der R¹ für einen aliphatischen Kohlenwasserstoffrest mit 5 bis 23 Kohlenstoffatomen und 0 oder 1 bis 5 Doppelbindungen steht,
mit einer solchen Menge einer Fettsäure der Formel **(II)**,
**R²-COOH (I)**
in der R² für einen aliphatischen Kohlenwasserstoffrest mit 11 bis 17 Kohlenstoffatomen und 0, 1 oder 2 Doppelbindungen steht,
als ausschließlichem sauren Katalysator versetzt, so daß sich im Gemisch - bezogen auf die gesamte Reaktionsmischung - eine Säurezahl von 15 bis 20 ergibt, und
b) das resultierende Gemisch bei erhöhter Temperatur und gegebenenfalls unter erhöhtem Druck mit niederen aliphatischen Alkoholen mit 1 bis 4 Kohlenstoffatomen einer Umesterung unterwirft.

2. Verfahren nach Anspruch 1, **dadurch gekennzeichnet**, daß man als niederen aliphatischen Alkohol Methanol einsetzt.

3. Verfahren nach den Ansprüchen 1 und 2, **dadurch gekennzeichnet**, daß man Triglyceride der Fettsäuren der Formel **(I)** einsetzt.

4. Verfahren nach mindestens einem der Ansprüche 1 bis 3, **dadurch gekennzeichnet**, daß man die Fettsäuren der Formel **(II)** in Mengen von 1 bis 20 Gew.-% - bezogen auf das Fettsäureglycerid - einsetzt.

5. Verfahren nach mindestens einem der Ansprüche 1 bis 4, **dadurch gekennzeichnet**, daß man die Umesterung bei Temperaturen von 150 bis 300°C durchführt.

6. Verfahren nach mindestens einem der Ansprüche 1 bis 5, **dadurch gekennzeichnet**, daß man die Umesterung bei Drücken von 1 bis 100 bar durchführt.

## Claims

1. A process for the production of fatty acid lower alkyl esters in which
a) a fatty acid corresponding to formula (II):
R²-COOH (II)
in which R² is an aliphatic hydrocarbon radical containing 11 to 17 carbon atoms and 0, 1 or 2 double bonds,
is added as sole acidic catalyst to
full and/or partial esters of glycerol with fatty acids corresponding to formula (I):
R¹-COOH (I)
in which R¹ is an aliphatic hydrocarbon radical containing 5 to 23 carbon atoms and 0 or 1 to 5 double bonds,
in such a quantity that an acid value of 15 to 20 is obtained in the mixture, based on the reaction mixture as a whole, and
b) the resulting mixture is transesterified with lower aliphatic alcohols containing 1 to 4 carbon atoms at elevated temperature and optionally under elevated pressure.

2. A process as claimed in claim 1, characterized in that methanol is used as the lower aliphatic alcohol.

3. A process as claimed in claims 1 and 2, characterized in that triglycerides of the fatty acids corresponding to formula (I) are used.

4. A process as claimed in at least one of claims 1 to 3, characterized in that the fatty acids corresponding to formula (II) are used in quantities of 1 to 20% by weight.

5. A process as claimed in at least one of claims 1 to 4, characterized in that the transesterification is carried out at temperatures of 150 to 300°C.

6. A process as claimed in at least one of claims 1 to 5, characterized in that the transesterification is carried out under pressures of 1 to 100 bar.

## Revendications

1. Procédé pour la préparation d'esters alkyliques inférieurs d'acides gras, dans lequel
a) on ajoute à des esters complets et/ou partiels de la glycérine avec des acides gras de formule (I)
R¹-COOH (I)
dans laquelle R¹ représente un radical d'hydrocarbure aliphatique contenant de 5 à 23 atomes de carbone et 0 ou de 1 à 5 doubles liaisons,
une quantité d'un acide gras de formule (II)
R²-COOH (II)
dans laquelle R² représente un radical d'hydrocarbure aliphatique contenant de 11 à 17 atomes de carbone et 0, 1 ou 2 doubles liaisons,
à titre de catalyseur acide exclusif, telle que l'on obtient dans le mélange - rapporté au mélange réactionnel total - un indice d'acide de 15 à 20, et
b) on soumet le mélange résultant à une transestérification à température élevée et éventuellement sous pression élevée avec des alcools aliphatiques inférieurs contenant de 1 à 4 atomes de carbone.

2. Procédé selon la revendication 1, caractérisé en ce qu'on met en oeuvre le méthanol comme alcool aliphatique inférieur.

3. Procédé selon les revendications 1 et 2, caractérisé en ce qu'on met en oeuvre des triglycérides des acides gras de formule (I).

4. Procédé selon au moins une des revendications 1 à 3, caractérisé en ce qu'on met en oeuvre les acides gras de formule (II) dans des quantités de 1 à 20% en poids - rapportées au glycéride d'acide gras.

5. Procédé selon au moins une des revendications 1 à 4, caractérisé en ce qu'on effectue la trans-estérification à des températures de 150 à 300°C.

6. Procédé selon au moins une des revendications 1 à 5, caractérisé en ce qu'on effectue la trans-estérification sous des pressions de 1 à 100 bar.
